# EUROPEAN PATENT SPECIFICATION

(11) **EP 1 732 875 B1**
(45) Date of publication and mention of the grant of the patent: **15.05.2013**
(21) Application number: 04734566.5
(22) Date of filing: 24.05.2004
(51) Int. Cl.: C07C 61/22

(54) **PROCESS FOR ISOLATION OF HEPATOPROTECTIVE AGENT "OLEANOLIC ACID" FROM LANTANA CAMARA**
VERFAHREN ZUR ISOLIERUNG DES LEBENSCHÜTZENDEN MITTELS OLEANOLSÄURE AUS LANTANA CAMARA
PROCEDE D'ISOLEMENT D'AGENT HEPATOPROTECTEUR "ACIDE OLEANOLIQUE" TIRE DE LANTANA CAMARA

(30) Priority: 30.03.2004 US 815095
(43) Date of publication of application: 20.12.2006
(73) Proprietor: Council of Scientific and Industrial Research, New Delhi 110 001 (IN)
(72) Inventor: SRIVASTAVA, Santosh K. Central Inst. of Medicinal, Utter Pradesh 226 015 (IN); KHAN, Merajuddin Central Institute of Medicinal &, Utter Pradesh 226 015 (IN); KHANUJA, Suman Preet Singh, Central Institute of, Uttar Pradesh 226 015 (IN)
(74) Representative: Srinivasan, Ravi Chandran
(86) International application number: PCT/IB2004/001676
(87) International publication number: WO 2005/095321

(56) References cited:
- DATABASE CHEMABS [Online] CHEMICAL ABSTRACTS SERVICE, COLUMBUS, OHIO, US; MISRA, LAXMI NARAIN ET AL: "A process for the extraction of oleanolic acid from Lantana camara" XP002297441 retrieved from STN Database accession no. 2004:317593 & IN 184 489 A (COUNCIL OF SCIENTIFIC AND INDUSTRIAL RESEARCH, INDIA) 26 August 2000 (2000-08-26)
- MISRA, L. ET AL: "Triterpenoids, essential oil and photo-oxidative 28,13-lactonization of oleanolic acid from Lantana camara" PHYTOCHEMISTRY , 54(8), 969-974 CODEN: PYTCAS; ISSN: 0031-9422, 2000, XP004291608
- MIRSA, L. N. ET AL: "High concentration of hepatoprotective oleanolic acid and its derivatives in Lantana camara roots" PLANTA MEDICA , 63(6), 582 CODEN: PLMEAA; ISSN: 0032-0943, 1997, XP009036804
- VERMA, D. K. ET AL: "Antimicrobial active triterpenoids from Lantana species" INDIAN DRUGS , 34(7), 390-392 CODEN: INDRBA; ISSN: 0019-462X, 1997, XP002115069

## Description

### FIELD OF INVENTION

The present invention relates to an improved and economical process for the isolation of hepatoprotective agent "oleanolic acid" from *Lantana camara.*

### BACKGROUND AND PRIOR ART

Oleanolic acid [(3-O-β-hydroxy-olea-12-en-28-oic acid is a triterpenoid compound, which exist widely in natural plants in the form of free acid or aglycones for triterpenoid saponins. Oleanolic acid has been isolated from more than 120 plant species. It has been identified as the main bioactive constituent of the medicinal plants used in folk medicine such as *Aralia chinensis,* var. nuda nakai, *Beta vulgaris* L. var. cicla L., *Swertia mileensis, Swertia japonica, Tetraponax papyriferum, Panax ginseng* used in hepatoprotection; *Ligustrum lucidum, Luffa cyclindrica, Oleandra neriifolia, Sapindus mulcorossi* used in anti-inflammation and *Gonoderma lucidum* and *Glechoma hederacea* used for anticarcinogenic activity and antitumor promotion. Oleanolic acid, as such has been reported to exhibit potent hepotoprotective activity. It decreases CCl₄-induced liver parenchymal cell necrosis, steatosis and degeneration plus alcohol-induced chronic cirrhosis. It is as such marketed in China for human hepatitis. Similarly oleanolic acid has also shown significant anti-inflammatory activity by inhibiting raw paw edema produced by dextran and by suppressing adjuvant induced arthritis in rats and mice. It is also important to note that oleanolic acid also inhibits tumor initiation and tumor promotion. Treatment of rats with oleanolic acid (200 ppm) in diet for 3 weeks decreases the incidence and multiplicity of azoxymethane-induced intestinal tumor. Oleanolic acid also showed significant hypolipidemic and anti-atherosclerotic properties. Treatment of experimental hyperlipidemic rats with oleanolic acid (50mg/kg, P.O. for 9 days) deceases the elevated blood cholesterol and β-lipoprotein levels by more than 40%. Cosmetic and pharmaceutical preparations of oleanolic have been patented in Japan for use in skin care and non-lymphatic leukemia.

Apart from the above, oleanolic acid has also shown antiulcer, antimicrobial, hypoglycaemic activity, protection against cyclophosphamide induced toxicity, anticarcinogenic and antifertility activities etc. It was observed that although oleanolic acid has been isolated from more than 120 plant species however, due to the poor yield and tedious column chromatographic separation procedures of the bioactive constituent from *Panax ginseng, Aralia chinensis, Eugenia Jaumbolana, Calendula officinalis, Gonoderma lucidum, Oleandra neriifolia* (Plants used in folk medicines) and most of the other plant species, this bioactive constituent has become an expensive pharmaceutical compound. This prompted us to search for an inexpensive, easily available, wildly growing and rich source of oleanolic acid and develop an easy and economical process for the isolation of this important therapeutic agent so that it can be brought under the reach of common masses.

On going through the literature, it was observed that sugar beets may be an inexpensive, easily available source of oleanolic acid. An extraction procedure for oleanolic acid has also been patented from Sugar beet ("Extraction of oleanolic acid from Sugar beets for treatment of liver failure", Yabuchi et al. 1988, chemical Abstract 108, 82082p; Yabuchi *et al.* 1987, Japanease pat. No. 62126149). This process involves crude oleanolic acid preparation from 1Kg each of sugar beet roots and leaves. For further purification, the crude preparations were extracted with MeOH, treated with HCl, and subjected to column chromatographic separation. The recovery rate from crude preparation was only 66.3%.

The method described above suffers from a number of disadvantages. The major disadvantage of the above method is the very low (exact yield not available) concentration of oleanolic acid saponin in both leaf and roots of sugar beets. The second disadvantage of the above process is that oleanolic acid has not been isolated as such, but was obtained after an extra and tedious acid hydrolysis step, which reduces the recovery of bioactive constituent by almost 33%. The third major disadvantage of the above process is that it utilizes column chromatographic separation for the isolation of oleanolic acid using various mixture of eluting solvents. Thus resulting in a tedious, time taking and expensive process for the isolation of oleanolic acid.

The other inexpensive, easily available and rich source of oleanolic acid is roots of *Lantana camera. L. camara* is a prickly climbing aromatic shrub of the family Verbenaceae. It is native to tropical America and was introduced in India as an ornamental and hedge plant, but now it has been completely naturalized and growing very wildly throughout India. A dense wild population of this shrub can be easily seen along the railway lines, forests and in almost all the wild places. It has also been recorded that different parts of the plant are rich source of various bioactive principles. In Africa, infusion of the leaves are used against rheumatism, asthama, cough and colds. The whole plant and its infusions are considered to be anti-pyretic, diaphoretic and anti-malarial. Recently an isolation procedure for oleanotic acid has been patented from the rootlets and root bark of *L*. *camara* ("High concentration of hepatoprotective oleanolic acid and its derivatives in Lantana camara roots", Misra et al 1997, Planta Med. 63: 582, Misra *et al.* 1996, *Indian Pat.* No.1.84489). This process involves extraction of rootlets and root bark with a mixture of three solvents. The crude so obtained is chromatographed on silica gel column and the oleanolic acid rich fractions are further purified on another column, thus resulting in the isolation of 1.47% of oleanolic acid.

The method described above suffers from a number of disadvantages- The biggest disadvantage of the above method is that it uses rootlets and root barks of *L*. *camara.* The rootlets are very small and few in *L. camara,* hence can not be obtained in sufficient amount for commercial purpose. Similarly the roots of *L. camara* are also small in size and are covered with very thin bark, hence peeling off the bark on commercial scale is neither possible nor it will be economical. It has been observed that rootlets and root barks in *L. camara* constitute not more than 20% of the total roots. Hence for obtaining 3.75Kg of rootlets and root bark, 18.75Kg of roots would have been certainly used, which gave only 55g of oleanolic acid in the above process. But if 18.75Kg of *L*. *camara* roots are processed according to our method it will give -187.5g of oleanolic acid. In this way it is very clear that the yield of olenolic acid by our process is 3.4 times more than the above process.

The second disadvantage of the above process is that it utilizes mixture of three solvents for the extraction of plant material, which neither can be used again nor can be recycled. The third major disadvantage of the above process is that it utilizes repeated column chromatography on silica gel for the isolation of oleanolic acid using mixtures of eluting solvents, thus resulting in a tedious, time taking and enormous expensive procedure, which can not be economically viable.

Vesma et al, Indiana drugs 34(7), July 1997, discloses a process in which roots and leaves of Lantana indicia and Lantana camora were dried, pulverised and extracted with chloroform, and in which the extract was purified by column chromatography.

### OBJECT OF THE INVENTION

The main object of the present invention is to provide an improved economical process for the isolation of oleanolic acid directly from the roots of *L. camara,* which obiates the draw backs of the existing processes.

Another object of the present invention is to completely avoid use of highly tedious, time taking column chomatographic purification process for the isolation of oleanolic acid from the roots of *Lantana camara.*

Still another object of the present invention is to provide an economical process for the isolation of oleanolic acid from the roots of *Lantana camara.*

Another object of the present invention is that it completely omit the use of highly tedious, time taking and expensive repeated column chromatographic purification process used in prior art processes.

Another object of the present invention is that it directly uses roots while the existing processes use only rootlets and root bark, resulting in a yield advantages of 3.4 times.

Another object of the present invention single solvent can be used for the extraction of oleanolic acid the plant material, which can be reused and/or recycled However, the prior art process uses mixture of three solvents, which can neither be reused nor can be recycled.

Still another object of the present invention is that this process uses simple precipitation and crystallization processes for the isolation of oleanolic acid which are easy, less time taking and highly inexpensive.

### SUMMARY OF THE INVENTION

The present application relates to a process according to the following embodiments
1. A process for the isolation of oleanolic acid from the roots of *Lantana camara,* said process comprising the steps:
   a) obtaining the dried root of *Lantana camara,*
   b) grinding the dried root of step (a) to obtain root powder,
   c) defattening the root powder for three times with an organic solvent for a time period in the range of 6-12 hours and at temperature in the range of 30-40°C,
   d) extracting the defattened root powder with a single solvent for three times at a temperature in the range of 30-40°C and for a time period in the range of 6 to 12 hours,
   e) removing the solvent used in step (d) to obtain a crude extract, and
   f) precipitating the crude extract to obtain a precipitate followed by repeated partial crystallization of the precipitate with a single solvent to obtain the oleanolic acid.
2. A process according to embodiment 1 wherein in steps (c), the solvents used are selected from a group comprising 95% ethyl alcohol, hexane, benzene, toluene and dichloromethane.
3. A process according to embodiment 1, step (d), the extraction is carried out with a single solvent selected from CH₂Cl₂, CHCl₃,EtOAc, ether, acetone, MeOH or EtOH.
4. A process as claimed in claim 1 wherein in step (e), the solvent removal is carried out under vacuum at a temperature in the range of 35 to 45°C.
5. A process according to embodiment 1 wherein in step (f), the precipitation of crude extract and repeated partial crystallization of precipitate is carried out with a single solvent selected from CH₂Cl₂, CHCl₃,EtOAc, ether, acetone, MeOH or EtOH
6. A process as claimed in claim 1, wherein the yield of olenolic acid is 1%.

Accordingly the present invention provides an improved and economical process for the isolation of oleanolic acid from the roots of *Lantana camera,* which comprises of drying, grinding and defattening of *Lantana camara* roots with light petroleum followed by over night extractions at room temperature (30-40°C) three times with a single solvent selected from CH₂Cl₂, CHCl₃, EtOAc, ether, acetone, MeOH, or EtOH, removal of solvent under vacuum at 35-45°C, precipitation of crude extract and repeated partial crystallization of precipitate with a single solvent selected from CH₂Cl₂, CHCl₃, EtOAc, ether, acetone, MeOH, EtOH, H₂O and others resulting in the isolation of oleanolic acid with 1% yield.

### DETAILED DESCRIPTION OF THE INVENTION

Accordingly the present invention provides an improved and economical process for the isolation of oleanolic acid from the roots of *Lantana camara,* which comprises of drying, grinding and defattening of *Lantana camara* roots with light petroleum followed by over night extractions et room temperature (30-40°C) three times with a single solvent selected from CH₂Cl₂, CHCl₃, EtOAc, ether, acetone, MeOH, or EtOH, removal of solvent under vacuum at 35-45°C, precipitation of crude extract and repeated partial crystallization of precipitate with a single solvent selected from CH₂Cl₂, CHCl₃ , EtOAc, ether, acetone, MeOH, EtOH, H₂O and others resulting in the isolation of oleanolic acid with 1% yield.

In an embodiment of the present invention a varied range of defattening solvents petroleum ether, hexane, benzene, toluene and dichloromethane can be used.

In another embodiment of the present invention a varied range of fractionating solvents CH₂Cl₂, CHCl₃, EtOAc, ether, actone, MeOH, and EtOH can be used.

Still in another embodiment of the present invention a varied range of precipitating and crystallizing solvents CH₂Cl₂, CH₃CHCl₂, CHCl₃, EtOAc, ether, actone, MeOH, EtOH and H₂O can be used.

In another embodiment of the present invention which completely omit the use of highly tedious, time taking and expensive repeated column chromatographic purification process used in prior art processes.

In another embodiment of the present invention which directly uses roots while the existing processes use only rootlets and root bark, resulting in a yield advantages of 3.4 times.

In another embodiment of the present invention wherein single solvent can be used for the extraction of oleanolic acid the plant material, which can be reused and/or recycled However, the prior art process uses mixture of three solvents, which can neither be reused nor can be recycled.

In another embodiment of the present invention uses simple precipitation and crystallization processes for the isolation of oleanolio acid which are easy, less time taking and highly inexpensive.

The following examples are given by way of illustration of the present invention.

### Example -1

The fresh *Lantana camara* roots were collected from the field. The roots were first washed and made free from soil and other organic matters. The clean roots were chopped into small pieces and shade dried. The dried roots were powdered in a grinder. The powdered *Lantana camara* roots (700g) were first hot defatted with petroleum ether (bp 40-60°C and then extracted with dichloromethane (CH₂Cl₂). The extraction was carried out for 8 hrs till the material was completely exhausted. Removal of the solvent under vacuum at 40°C gave a brownish viscous mass. This was dissolved in excess of water and left over night at room temperature. The precipitate so obtained was filtered and the precipitate was crystallized with ether four times, which resulted in the isolation of oleanolic acid in 0.7% yield.

### Example - 2

The powdered roots (2Kg) were first cold defatted with hexane and then extracted with MeOH over night four times at room temperature. Removal of the solvent was carried out under vacuum at 40°C. The crude extract was dissolved in excess of EtOAC and left overnight at room temperature. The precipitate was filtered and crystallized with MeOH. Precipitation and crystallization processes were repeated 4 times, which resulted in the isolation of oleanolic acid in 0.85% yield.

### Example - 3

The powdered roots (1.5Kg) were first hot defatted with petroleum ether (bp 40-60°C) and then extracted with acetone. The extraction areas carried out for about 8 hrs till the material was completely exhausted. Removal of the solvent under vacuum at 40°C gave a brownish viscous mass. This was dissolved in excess of dichloromethane by heating and then left over night at room temperature. The precipitate so obtained was filtered and crystallized with ether thrice, which resulted in the isolation of oleanolic acid in 0.65% yield.

### Example 4

The powdered roots (3Kg) were first cold defatted thrice with petroleum ether (bp 40-60°C) at room temperature over night. The defatted material was then extracted with CHCl₃ four times at room temperature over night. Removal of the solvent was carried out under vacuum at 40°C. The crude extract so obtained was dissolved in acetone and left over night for precipitation. The precipitate so obtained was filtered and crystallized with EtOH. Precipitation and crystallization process were repeated 4 times which gave oleanolic acid in 0.8% yield.

### Example - 5

The powdered roots of *L. camara* (5Kg) were defatted with hexane in cold thrice at room temperature. The defatted material was then extracted with EtOAc four times overnight at room temperature. The solvent was removed under vacuum at 40°C and the crude extract so obtained was dissolved in ether and left over night in refrigerator for precipitation. The precipitate so obtained was filtered and dissolved in MeOH for crystallization. Precipitation and crystallization process were repeated for 4 times, which gave oleanolic acid in 0.7% yield.

### Example -6

The powdered roots of *L. camara* (10Kg) were defatted thrice in cold overnight with petroleum ether (bp 40-60°C) and then extracted exhaustively with EtOH four times overnight at room temperature. The solvent was removed under vacuum at 40°C and the crude was dissolved in CHCl₃ and left overnight for precipitation. The precipitate so obtained was crystallized with MeOH. Precipitation and crystallization process were repeated 4 times, which gave oleanolic acid in 0.9% yield.

### Advantages

1. The main advantage of our process is that it completely omit the use of highly tedious, time taking and expensive repeated column chromatographic purification process used in prior art processes.
2. The other major advantage of our process is that it directly uses roots while the existing processes use only rootlets and root bark, resulting in a yield advantages of 3.4 times.
3. Single solvent can be used for the extraction of oleanolic acid the plant material, which can be reused and/or recycled However, the prior art process uses mixture of three solvents, which can neither be reused nor can be recycled.
4. The present process uses simple precipitation and crystallization processes for the isolation of oleanolic acid which are easy, less time taking and highly inexpensive.

## Claims

1. A process for the isolation of oleanolic acid from the roots of *Lantana camara,* said process comprising the steps:
a) obtaining the dried root of *Lantana camara.*
b) grinding the dried root of step (a) to obtain root powder,
c) defattening the root powder for three times with an organic solvent for a time period in the range of 6-12 hours and at temperature in the range of 30-40°C,
d) extracting the defattened root powder with a single solvent for three times at a temperature in the range of 30-40°C and for a time period in the range of 6 to 12 hours,
e) removing the solvent used in step (d) to obtain a crude extract, and
f) precipitating the crude extract to obtain a precipitate followed by repeated partial crystallization of the precipitate with a single solvent to obtain the oleanolic acid.

2. A process as claimed in claim 1 wherein in steps (c), the solvents used are selected from a group comprising 95% ethyl alcohol, hexane, benzene, toluene and dichloromethane.

3. A process as claimed in claim 1, step (d), the extraction is carried out with a single solvent selected from CH₂Cl₂, CHCl₃,EtOAc, ether, acetone, MeOH or EtOH.

4. A process as claimed in claim 1 wherein in step (e), the solvent removal is carried out under vacuum at a temperature in the range of 35 to 45°C.

5. A process as claimed in claim 1 wherein in step (f), the precipitation of crude extract and repeated partial crystallization of precipitate is carried out with a single solvent selected from CH₂Cl₂, CHCl₃,EtOAc, ether, acetone, MeOH or EtOH

6. A process as claimed in claim 1, wherein the yield of olenolic acid is 1%.

## Patentansprüche

1. Verfahren zur Isolierung von Oleanolsäure aus den Wurzeln von *Lantana camara,* wobei das Verfahren die Schritte:
a) Erhalten der getrockneten Wurzel von *Lantana camara,*
b) Mahlen der getrockneten Wurzel von Schritt (a) unter Erhalt von Wurzelpulver,
c) dreimaliges Entfetten des Wurzelpulvers mit einem organischen Lösungsmittel für einen Zeitraum im Bereich von 6-12 Stunden und bei einer Temperatur im Bereich von 30-40°C,
d) Extrahieren des entfetteten Wurzelpulvers dreimal mit einem einzelnen Lösungsmittel bei einer Temperatur im Bereich von 30-40°C und für einen Zeitraum im Bereich von 6 bis 12 Stunden,
e) Entfernen des in Schritt (d) verwendeten Lösungsmittels unter Erhalt eines rohen Exraktes und
f) Präzipitieren des rohen Extraktes unter Erhalt eines Präzipitats, gefolgt von wiederholter partieller Kristallisation des Präzipitats mit einem einzelnen Lösungsmittel, um die Oleanolsäure zu erhalten,
umfasst.

2. Verfahren, wie es in Anspruch 1 beansprucht ist, wobei in Schritt (c) die verwendeten Lösungsmittel aus der Gruppe, umfassend 95% Ethylalkohol, Hexan, Benzol, Toluol und Dichlormethan, ausgewählt werden.

3. Verfahren, wie es in Anspruch 1 beansprucht ist, wobei Schritt (d), die Extraktion, mit einem einzelnen Lösungsmittel, ausgewählt aus CH₂Cl₂, CHCl₃, EtOAc, Ether, Aceton, MeOH oder EtOH, durchgeführt wird.

4. Verfahren, wie es in Anspruch 1 beansprucht ist, wobei in Schritt (e) die Lösungsmittelentfernung unter Vakuum bei einer Temperatur im Bereich von 35 bis 45°C durchgeführt wird.

5. Verfahren, wie es in Anspruch 1 beansprucht wird, wobei in Schritt (f) die Präzipitation von rohem Extrakt und eine wiederholte partielle Kristallisation von Präzipitat mit einem einzelnen Lösungsmittel, ausgewählt aus CH₂Cl₂, CHCl₃, EtOAc, Ether, Aceton, MeOH oder EtOH, durchgeführt wird.

6. Verfahren, wie es in Anspruch 1 beansprucht ist, wobei die Ausbeute an Oleanolsäure 1% beträgt.

## Revendications

1. Procédé permettant d'isoler de l'acide oléanolique à partir de racines de *Lantana camara,* lequel procédé comporte les étapes suivantes :
a) se procurer des racines séchées de *Lantana camara,*
b) broyer les racines séchées de l'étape (a), pour en faire une poudre de racines,
c) dégraisser cette poudre de racines à trois reprises avec un solvant organique, durant un laps de temps de 6 à 12 heures et à une température de 30 à 40 °C,
d) soumettre cette poudre de racines dégraissée à une opération d'extraction réalisée à trois reprises avec un seul solvant, à une température de 30 à 40 °C et durant un laps de temps de 6 à 12 heures,
e) chasser le solvant utilisé dans l'étape (d) pour obtenir un extrait brut,
f) et faire précipiter cet extrait brut de manière à obtenir un précipité, et faire ensuite cristalliser partiellement et de façon répétée ce précipité, à l'aide d'un seul solvant, pour obtenir de l'acide oléanolique.

2. Procédé conforme à la revendication 1, pour lequel, dans les opérations de l'étape (c), les solvants utilisés sont choisis dans l'ensemble constitué par les suivants : alcool éthylique à 95 %, hexane, benzène, toluène et dichlorométhane.

3. Procédé conforme à la revendication 1, dans lequel, dans l'étape (d), on effectue l'extraction avec un seul solvant choisi parmi les dichlorométhane, chloroforme, acétate d'éthyle, éther, acétone, éthanol et méthanol.

4. Procédé conforme à la revendication 1, dans lequel, dans l'étape (e), on effectue l'élimination du solvant en opérant sous vide et à une température de 35 à 45 °C.

5. Procédé conforme à la revendication 1, dans lequel, dans l'étape (f), les opérations de formation d'un précipité d'extrait brut et de cristallisations partielles répétées de ce précipité sont effectuées avec un seul solvant choisi parmi les dichlorométhane, chloroforme, acétate d'éthyle, éther, acétone, éthanol et méthanol.

6. Procédé conforme à la revendication 1, dont le rendement en acide oléanolique vaut 1 %.
